# EUROPEAN PATENT SPECIFICATION

(11) **EP 1 489 981 B1**
(45) Date of publication and mention of the grant of the patent: **25.02.2009**
(21) Application number: 03713163.8
(22) Date of filing: 02.04.2003
(51) Int. Cl.: A61B 17/72, A61F 2/30

(54) **MEDULLARY NAIL FOR FIXATION OF BONE FRAGMENTS AT BONE FRACTURES**
MARKNAGEL ZUR FIXIERUNG VON KNOCHENFRAGMENTEN BEI KNOCHENFRAKTUREN
CLOU MEDULLAIRE PERMETTANT DE FIXER DES FRAGMENTS D'OS AU NIVEAU DE FRACTURES OSSEUSES

(30) Priority: 04.04.2002 SE 0201011
(43) Date of publication of application: 29.12.2004
(73) Proprietor: Hansson, Henrik, 590 77 Vreta Kloster (SE)
(72) Inventor: Hansson, Henrik, 590 77 Vreta Kloster (SE)
(74) Representative: Wagner, Karl Heinz
(86) International application number: PCT/SE2003/000524
(87) International publication number: WO 2003/084416

(56) References cited:
- EP-A2- 0 853 931
- US-A- 5 620 445

## Description

The present invention relates to a medullary nail for fixation of bone fragments at bone fractures, wherein the medullary nail is adapted to be inserted into holes in medullary canals in the bone fragments, and wherein front parts of the medullary nail are adapted to be locked to one of the bone fragments by means of a locking means.

In order to fix the front members of a medullary nail at a bone fragment, one can use a locking means e.g. in the form of a screw which is screwed into a predrilled, transverse hole in the bone fragment and through a transverse hole in said front parts of the medullary nail.

When screwing the screw into the bone fragment, said screw often ends up obliquely relative to the transverse hole in the medullary nail, and for being able also in such cases to screw said screw through said hole, said hole must have a substantially larger or at least a great deal larger diameter than the screw. This means however, that there will be a play between the medullary nail and the screw so that the medullary nail can move particularly in torsional directions but also in axial directions relative to the screw. This in turn, means that the bone fragments will be able to move relative to each other, i.e. the fixation will not be stable.

It is also very difficult to hit the hole in the medullary nail with a drill by means of which the transverse hole is drilled. This is done during radioscopy and it is very common that the surgeon is forced to drill several times on different locations, which results in a strongly weakened bone, while simultaneously the surgeon and the patient are subjected to radiation for an unsuitable long time.

On ulna, radius and fibula, it is not possible to use conventional medullary nails and securing said nails with transverse screws, because the medullary canal in these bones is too small for being able to use medullary nails having sufficiently large diameter for providing them with the required large hole for a transverse screw.

In EP-A2-0 853 931, the terminal element 13 is provided, through a snap-in action, in slots 3 at the edge 5 of a distal terminal element of a stem 1 of a primary hip prosthesis in order to flare out the sectors 4 thereof and thus lock the primary hip prosthesis. The prior art terminal element 13 does not in any way engage any bone fragment. It is used only to flare out said sectors 4, via said snap-in action, and maintain this widening of the sectors 4 for locking the prosthesis in the medullary canal C. The terminal element 13 however, is not fixed relative to the bone and the locking of the medullary nail will therefore be inferior and unreliable.

The object of the present invention is to remedy said problems and accomplish that the medullary nail can be fixed substantially immovable at the locking means. This is arrived at by providing the device according to the invention with the characterizing features of subsequent claim 1.

Since the medullary nail has a snap-in device, allowing said nail to be snapped-in onto the locking means located in a transverse hole in one of the bone fragments, it is accomplished that the front members or parts of the medullary nail can be fixed substantially immovably at the locking means. Furthermore, the snap-in device can be designed such that the locking means easily captures the medullary nail when said nail is threaded into the medullary canal.

The invention will be further described below with reference to the accompanying drawing, in which
figure 1 is a longitudinal vertical section of a forearm bone with a medullary nail according to the invention; and
figures 2, 3 and 4 illustrate a longitudinal horizontal section of front parts of the medullary nail of figure 1 in different positions relative to a locking means during a fixation movement at which the medullary nail is fixed at the locking means.

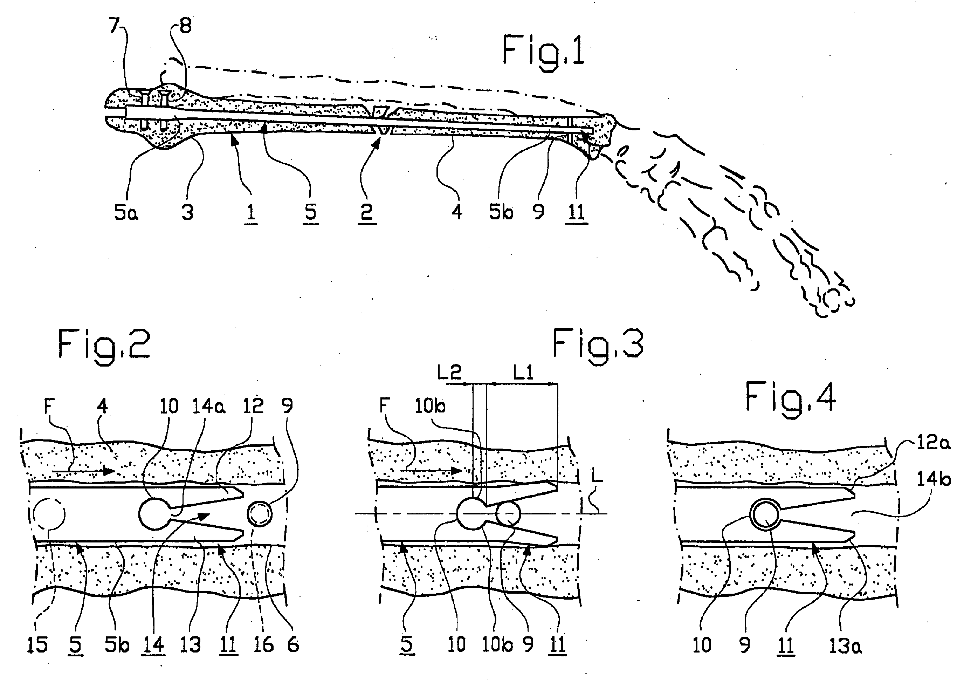

The forearm bone 1 (ulna) illustrated with a section in figure 1 has a bone fracture 2 at which the forearm bone 1 is broken in two or more bone fragments, e.g. an upper bone fragment 3 and a lower bone fragment 4. These bone fragments 3, 4 are fixed relative to each other by means of a medullary nail 5 which is inserted in a hole 6 which has been drilled in the longitudinal direction in the medullary canals of the bone fragments 3, 4. In the upper bone fragment 3, a number of holes, e.g. two transverse holes, have been drilled for two transverse locking screws 7, 8 or corresponding locking means which extend through two transverse holes in rear parts 5a of the medullary nail 5. The transverse locking screws 7, 8 are adapted to fix the upper bone fragment 3 and the rear parts 5a of the medullary nail 5 relative to each other. In the lower bone fragment 4 a transverse hole has been drilled into which a transverse locking screw 9 is screwed. This locking screw 9 is adapted to extend through a transverse hole 10 in front parts 5b of the medullary nail 5. The locking screw 9 is adapted to fix the lower bone fragment 4 and the front parts 5b of the medullary nail 5 relative to each other and it can be extracted from the hole 10 by being backed out therefrom and from the hole therefor in the lower bone fragment 4.

The instrument for drilling a longitudinal hole in the medullary canal for medullary nails 5 and transverse holes for locking screws, are commonly known and therefor not further described.

The front parts 5b of the medullary nail 5 has a snap-in device 11 for attaching by snap-in action said front parts 5b to the transverse locking screw 9. The snap-in device 11 is designed to be threaded onto the locking screw 9 by moving it in a forward direction in the hole 6 in the medullary canal as is shown with an arrow F in figures 2 and 3. Hereby, the snap-in device 11 is brought to be opened by the locking screw 9 - as is apparent from figure 3 - and when the locking screw 9 is situated in the hole 10, the snap-in device 11 will snap--in to its closed condition - as is shown in figure 4 - which means that the snap-in device 11 retains the medullary nail 5 at the locking screw 9.

The snap-in-device 11 is preferably designed such that it can hold the medullary nail 5 at the locking screw 9 with a firm grip such that the medullary nail 5 can move neither in axial direction relative to its longitudinal axis L nor rotate relative thereto.

The snap-in device 11 is also preferably designed such that it can not loosen or be drawn away from the locking screw 9.

The snap-in device 11 can be designed in different ways for obtaining the abovementioned snap-in result at the locking screw 9. The snap-in device 11 illustrated in the drawings has two jaws 12, 13 which define between them a gap 14 which is open in forward direction and of which inner parts 14a are open towards the hole 10. Said inner parts 14a of the gap 14 has a smaller width than the hole 10 and the diameter of the locking screw 9. The jaws 12, 13 are elastic such that the locking screw 9 brings them to spring in a direction from each other when the snap-in device 11 is threaded thereon. When the snap-in device 11 has been threaded so far onto the locking screw 9 that said screw is situated in the hole 10, the jaws 12, 13 have sprung back to their starting positions due to their elasticity, whereby said jaws 12, 13 retain the medullary nail 5 on the locking screw 9.

The jaws 12, 13 can be provided such that the gap 14 tapers successively in a direction towards the inner parts 14a thereof and to the hole 10. The jaws may further be provided such that outer parts 14b of the gap 14 are rather much wider than the diameter of the locking screw 9, which means that the snap-in device 11 is easier oriented (if necessary by rotating the medullary nail 5 somewhat about its longitudinal axis L) relative to the locking screw 9 when said device shall be threaded onto said screw.

In the embodiment shown, the snap-in device 11 has a keyhole like shape defined by the gap 14 between the jaws 12, 13 and the hole 10. Seen in the longitudinal direction of the medullary nail 5, the jaws 12, 13 have a substantially greater length L1 than the sides 10a, 10b, of the hole 10 closest to the gap 14 and/or the inner sides of the jaws 12, 13 form smaller angles with the geometric longitudinal axis L of the medullary nail 5, than front portions of said sides 10a, 10b. This means that substantially less compressive forces are needed for pressing the medullary nail 5 on the locking screw 9 than for pulling off said medullary nail 5 from said screw. If the inner parts 14a of the gap 14 are substantially narrower than the diameter of the hole 10, it may further be almost impossible to remove the medullary nail 5 from the locking screw 9 unless exceptionally large pulling forces are used.

The locking screw 9 can be replaced by another locking means. The medullary nail 5 may in a manner known per se consist of a metallic material and may further be designed in a known manner. The snap-in device 11 of the medullary nail 5 may have jaws 12, 13 with bevelled outer edge portions 12a, 13a.

The medullary nail 5 may be designed for use at fractures on other bones that an fractures on forearm bones 1. Thus, the medullary nail 5 may e.g. be designed for use at all fractures on tube bones, e.g. radius, fibula, fibia, femur or humerus.

The front parts 5b of the medullary nail 5 may eventually have a second transverse hole 15 behind the hole 10 (shown with broken lines in figure 2). The locking screw 9 may have a hole 16 (shown with broken lines in figure 2) or another attachment member for attaching a fixture (not shown) to the locking screw 9. This fixture can be adapted to guide a drill for drilling a second hole for a second transverse locking screw (not shown), which shall be screwed through the hole 15.

The medullary nail 5 may eventually be cannulated for threading onto a guide applied in the bone.

## Claims

1. Medullary nail for fixation of bone fragments at bone fractures,
wherein the medullary nail (5) is adapted to be inserted into holes (6) in medullary canals in the bone fragments (3, 4), and
wherein front parts (5b) of the medullary nail (5) are adapted to be locked to one of the bone fragments (3, 4) by means of a locking means (9)
**characterized in**
**that** the locking means (9) for locking the front parts (5b) of the medullary nail to one of the bone fragments (3, 4) is adapted to be located in a transverse hole in the bone fragment and to extend through a transverse hole (10) in said front parts (5b) of the medullary nail (5), and
**that** the front parts (5b) of the medullary nail (5) includes a snap-in device (11) which is adapted to be threaded onto the locking means (9) and thereby opened by the locking means (9) and to be snapped-in again to a closed condition when the locking means (9) is located in the transverse hole (10) in the front parts (5b) of the medullary nail (5) such that the snap-in device (11) retains the medullary nail (5) at the locking means (9).

2. Medullary nail according to claim 1, **characterized in that** the snap-in device (11) is designed to retain the medullary nail (5) in a firm grip at the locking means (9).

3. Medullary nail according to claim 2, **characterized in that** the snap-in device (11) is designed to retain the medullary nail (5) at the locking means (9) such that the medullary nail (5) can move neither substantially in axial direction relative to the longitudinal axis thereof nor rotate in relation thereto.

4. Medullary nail according to any preceding claim, **characterized in that** the snap-in device (11) is designed such that the medullary nail (5) comprises means preventing the nail from loosering or being pulled off from the locking means (9).

5. Medullary nail according to any preceding claim, **characterized in that** the snap-in device (11) has two jaws (12, 13) which are provided such that the locking means (9), when the medullary nail (5) is moved in a direction (F) in parallel with its longitudinal axis (L) towards said locking means (9), is located between said jaws (12, 13), eventually after rotation of the medullary nail (5) about said longitudinal axis (L) if necessary.

6. Medullary nail according to any preceding claim, **characterized in**
**that** said two jaws (12, 13) define a gap (14) of which inner parts (14a) are open towards the transverse hole (10) in the front parts (5b) of the medullary nail (5),
**that** the inner parts (14a) of the gap (14) have a less width than the diameter of the transverse hole (10) and a less width than the width or diameter of the locking means (9),
**that** the jaws (12, 13) are elastic such that the locking means (9) brings said jaws to spring apart when the snap-in device (11) is threaded onto said locking means, and
**that** the jaws (12, 13) due to their elasticity can spring back to their original positions when the snap-in device (11) has been threaded so far onto the locking means (9) that said locking means (9) is situated in the transverse hole (10), whereby the snap-in device (11) retains the medullary nail (5) on the locking means (9).

7. Medullary nail according to claim 6, **characterized in that** the jaws (12, 13) are provided such that the gap (14) tapers in a direction towards its parts (14a) and to the transverse hole (10).

8. Medullary nail according to any of claims 6 or 7, **characterized in that** the jaws (12, 13) are provided such that outer parts (14b) of the gap (14) are wider than the locking means (9).

9. Medullary nail according to any of claims 6-8, **characterized in**
**that** the jaws (12, 13) have a substantially greater length (L1) than sides (10a, 10b) of the hole (10) closest to the gap (14) seen in the longitudinal direction of the medullary nail (5), and/or
**that** inner sides of the jaws (12, 13) define or form smaller angles with a geometric longitudinal axis (L) of the medullary nail (5) than front portions of sides (10a, 10b) of the hole (10) closest to the gap (14).

10. Medullary nail according to any of claims 6-9, **characterized in that** the jaws (12, 13) have bevelled outer edge portions (12a, 13a).

11. Medullary nail according to any preceding claim, **characterized in that** the locking means (9) is a locking screw which is screwed into the hole in a bone fragment (4) and which can be removed or extracted from the transverse hole (10) in the front parts (5b) of the medullary nail (5) by being unscrewed or backed out of said hole in the bone fragment (4).

12. Medullary nail according to any preceding claim, **characterized in**
**that** the front parts (5b) of the medullary nail (5) has a second transverse hole (15) behind said first transverse hole (10), and
**that** the transverse locking means (9) has a hole (16) or another attaching member for attachment of a fixture thereto, said fixture being adapted to guide a drill for drilling a second hole in a lower bone fragment (4) for a second transverse locking means which is adapted to extend through the second transverse hole (15) of the medullary nail (5).

13. Medullary nail according to any preceding claim, **characterized in that** said medullary nail (5) is adapted to be used for forearm bones, e.g. ulna.

## Patentansprüche

1. Marknagel zur Fixierung von Knochenfragmenten bei Knochenfrakturen,
bei dem der Marknagel (5) für die Einführung in Löcher (6) in Markkanälen in den Knochenfragmenten (3, 4) ausgelegt ist, und
bei dem vordere Abschnitte (5b) des Marknagels (5) dafür ausgelegt sind, dass sie mit Hilfe eines Arretierungsmittels (9) an einem der Knochenfragmente (3, 4) arretiert werden können,
**dadurch gekennzeichnet,**
**dass** das Arretierungsmittels (9) zum Arretieren der vorderen Abschnitte (5b) des Marknagels an einem der Knochenfragmente (3, 4) so ausgelegt ist, dass es in einem quer verlaufenden Loch im Knochenfragment positioniert werden kann und sich durch ein quer verlaufendes Loch (10) in die vorderen Abschnitte (5b) des Marknagels (5) erstreckt, und
**dass** die vorderen Abschnitte (5b) des Marknagels (5) eine Rastvorrichtung (11) aufweisen, die dafür ausgelegt ist, dass sie auf das Arretierungsmittel (9) aufgefädelt und **dadurch** durch das Arretierungsmittel (9) geöffnet werden kann und in einen geschlossenen Zustand zurückrasten kann, wenn sich das Arretierungsmittel (9) so im quer verlaufenden Loch (10) in den vorderen Abschnitten (5b) des Marknagels (5) befindet, dass die Rastvorrichtung (11) den Marknagel (5) im Arretierungsmittel (9) festhält.

2. Marknagel gemäß Anspruch 1, **dadurch gekennzeichnet, dass** die Rastvorrichtung (11) so ausgelegt ist, dass sie den Marknagel (5) mit festem Griff am Arretierungsmittel (9) festhält.

3. Marknagel gemäß Anspruch 2, **dadurch gekennzeichnet, dass** die Rastvorrichtung (11) so ausgelegt ist, dass sie den Marknagel (5) so im Arretierungsmittel (9) festhält, dass der Marknagel (5) sich weder im Wesentlichen in axialer Richtung zu seiner Längsachse bewegen kann noch relativ zu dieser Achse drehen kann.

4. Marknagel gemäß einem der vorhergehenden Ansprüche, **dadurch gekennzeichnet, dass** die Rastvorrichtung (11) so ausgelegt ist, dass der Marknagel (5) Mittel umfasst, die verhindern, dass der Nagel sich lockert oder aus dem Arretierungsmittel (9) weggezogen wird.

5. Marknagel gemäß einem der vorhergehenden Ansprüche, **dadurch gekennzeichnet, dass** die Rastvorrichtung (11) zwei Klemmbacken (12, 13) besitzt, die so vorgesehen ist, dass, wenn der Marknagel (5) in einer Richtung (F) parallel zu seiner Längsachse (L) auf das Arretierungsmittel (9) zu bewegt wird, sich das Arretierungsmittel (9) zwischen den Klemmbacken (12, 13) befindet, eventuell nach Drehung des Marknagels (5) um die Längsachse (L), wenn nötig.

6. Marknagel gemäß einem der vorhergehenden Ansprüche,
**dadurch gekennzeichnet,**
**dass** die Rastvorrichtung (11) zwei Klemmbacken (12, 13) besitzt, die eine Lücke (14) begrenzen, deren innere Abschnitte (14a) zum quer verlaufenden Loch (10) in den vorderen Abschnitten (5b) des Marknagels (5) hin offen sind,
**dass** die inneren Abschnitte (14a) der Lücke (14) weniger breit sind als der Durchmesser des quer verlaufenden Lochs (10) und weniger breit als die Breite oder der Durchmesser des Arretierungsmittels (9),
**dass** die Klemmbacken (12, 13) elastisch sind, so dass das Arretierungsmittel (9) die Klemmbacken dazu bringt, auseinander zu federn, wenn die Rastvorrichtung (11) auf das Arretierungsmittel aufgefädelt wird, und
**dass** die Klemmbacken (12, 13) aufgrund ihrer Elastizität in ihre ursprünglichen Positionen zurückfedern können, wenn die Rastvorrichtung (11) so weit auf das Arretierungsmittel (9) aufgefädelt worden ist, dass sich das Arretierungsmittel (9) im quer verlaufenden Loch (10) befindet, wodurch die Rastvorrichtung (11) den Marknagel (5) im Arretierungsmittel (9) festhält.

7. Marknagel gemäß Anspruch 6, **dadurch gekennzeichnet, dass** die Klemmbacken (12, 13) so vorgesehen sind, dass sich die Lücke (14) in einer Richtung auf ihre Abschnitte (14a) und auf das quer verlaufende Loch (10) zu verjüngt.

8. Marknagel gemäß Anspruch 6 oder 7, **dadurch gekennzeichnet, dass** die Klemmbacken (12, 13) so vorgesehen sind, dass äußere Abschnitte (14b) der Lücke (14) breiter sind als das Arretierungsmittel (9).

9. Marknagel gemäß einem der Ansprüche 6 - 8,
**dadurch gekennzeichnet,**
**dass** die Klemmbacken (12, 13) eine im Wesentlichen größere Länge (L1) haben als die Seiten (10a, 10b) des Lochs (10) unmittelbar neben der Lücke (14), in Längsrichtung des Marknagels (5) gesehen, und/oder
**dass** innere Seiten der Klemmbacken (12, 13) kleinere Winkel zu einer geometrischen Längsachse (L) des Marknagels (5) einschließen oder bilden als vordere Abschnitte der Seiten (10a, 10b) des Lochs (10) unmittelbar neben der Lücke (14).

10. Marknagel gemäß einem der Ansprüche 6 - 9, **dadurch gekennzeichnet, dass** die Klemmbacken (12, 13) abgeschrägte äußere Kantenabschnitte (12a, 13a) haben.

11. Marknagel gemäß einem der vorhergehenden Ansprüche, **dadurch gekennzeichnet, dass** das Arretierungsmittel (9) eine Sicherungsschraube ist, die in das Loch in einem Knochenfragment (4) eingeschraubt wird und durch Herausschrauben oder Zurückziehen aus dem Loch im Knochenfragment (4) aus dem quer verlaufenden Loch (10) in den vorderen Abschnitten (5b) des Marknagels (5) entfernt oder herausgezogen werden kann.

12. Marknagel gemäß einem der vorhergehenden Ansprüche,
**dadurch gekennzeichnet,**
**dass** die vorderen Abschnitte (5b) des Marknagels (5) hinter dem ersten quer verlaufenden Loch (10) ein zweites quer verlaufendes Loch (15) haben, und dass das quer verlaufende Arretierungsmittel (9) ein Loch (16) oder ein anderes Befestigungselement, an dem eine Vorrichtung befestigt werden kann, hat, wobei die Vorrichtung ausgelegt ist für die Führung eines Bohrers zum Bohren eines zweiten Lochs in einem unteren Knochenfragment (4) für ein zweites quer verlaufendes Arretierungsmittel, das dafür ausgelegt ist, dass es sich durch das zweite quer verlaufende Loch (15) des Marknagels (5) erstreckt.

13. Marknagel gemäß einem der vorhergehenden Ansprüche, **dadurch gekennzeichnet, dass** der Marknagel (5) zur Verwendung für Unterarmknochen, z.B. die Elle, ausgelegt ist.

## Revendications

1. Clou médullaire pour la fixation de fragments d'os à des fractures osseuses,
dans lequel le clou médullaire (5) est conçu pour venir s'insérer dans des trous (6) pratiqués dans des canaux médullaires dans les fragments d'os (3, 4), et
dans lequel les parties frontales (5b) du clou médullaire (5) sont conçues pour être verrouillées à un des fragments d'os (3, 4) à l'aide d'un moyen de verrouillage (9) ;
**caractérisé**
**en ce que** le moyen de verrouillage (9) pour verrouiller les parties frontales (5b) du clou médullaire à un des fragments d'os (3, 4) est conçu pour venir se disposer dans un trou transversal pratiqué dans le fragment d'os et pour s'étendre à travers un trou transversal (10) pratiqué dans lesdites parties frontales (5b) du clou médullaire (5) ; et
**en ce que** les parties frontales (5b) du clou médullaire (5) englobent un dispositif à enclenchement rapide (11) qui est conçu pour venir se visser sur le moyen de verrouillage (9) et pour ainsi être ouvert par le moyen de verrouillage (9) et qui est conçu pour se refermer par réenclenchement rapide lorsque le moyen de verrouillage (9) est disposé dans le trou transversal (10) pratiqué dans les parties frontales (5b) du clou médullaire (5) de telle sorte que le dispositif d'enclenchement rapide (11) retient le clou médullaire (5) au moyen de verrouillage (9).

2. Clou médullaire selon la revendication 1, **caractérisé en ce que** le dispositif d'enclenchement rapide (11) est conçu pour retenir le clou médullaire (5) fermement serré au moyen de verrouillage (9).

3. Clou médullaire selon la revendication 2, **caractérisé en ce que** le dispositif d'enclenchement rapide (11) est conçu pour retenir le clou médullaire (5) au moyen de verrouillage (9) de telle sorte que le clou médullaire (5) ne peut ni se déplacer essentiellement en direction axiale par rapport à son axe longitudinal, ni tourner par rapport à ce dernier.

4. Clou médullaire selon l'une quelconque des revendications précédentes, **caractérisé en ce que** le dispositif d'enclenchement rapide (11) est conçu de telle sorte que le clou médullaire (5) comprend un moyen pour empêcher le clou de se desserrer ou de s'écarter du moyen de verrouillage (9) par traction.

5. Clou médullaire selon l'une quelconque des revendications précédentes, **caractérisé en ce que** le dispositif d'enclenchement rapide (11) possède deux mâchoires (12, 13) qui sont prévues de telle sorte que le moyen de verrouillage (9), lorsque le clou médullaire (5) est déplacé dans la direction (F) parallèlement à son axe longitudinal (L) en direction dudit moyen de verrouillage (9), est disposé entre lesdites mâchoires (12, 13), en fin de compte, après rotation du clou médullaire (5), autour dudit axe longitudinal (L), si nécessaire.

6. Clou médullaire selon l'une quelconque des revendications précédentes, **caractérisé**
**en ce que** le dispositif d'enclenchement rapide (11) possède deux mâchoires (12, 13) définissant un espace libre (14) dont les parties internes (14a) sont ouvertes sur le trou transversal (10) pratiqué dans les parties frontales (5b) du clou médullaire (5) ;
**en ce que** la largeur des parties internes (14a) de l'espace libre (14) est inférieure au diamètre du trou transversal (10) et est inférieure à la largeur ou au diamètre du moyen de verrouillage (9) ;
**en ce que** les mâchoires (12, 13) sont élastiques de telle sorte que le moyen de verrouillage (9) fait s'écarter lesdites mâchoires l'une de l'autre par déclic lorsque le dispositif d'enclenchement rapide (11) est vissé sur ledit moyen de verrouillage ; et
**en ce que** les mâchoires (12, 13), grâce à leur élasticité, peuvent reprendre leurs positions initiales par déclic lorsque le dispositif d'enclenchement rapide (11) a été vissé à ce point sur le moyen de verrouillage (9) que ledit moyen de verrouillage (9) est situé dans le trou transversal (10), le dispositif d'enclenchement rapide (11) retenant ainsi le clou médullaire (5) sur le moyen de verrouillage (9).

7. Clou médullaire selon la revendication 6, **caractérisé en ce que** les mâchoires (12, 13) sont prévues de telle sorte que l'espace libre (14) se rétrécit dans une direction s'étendant vers ses parties (14a) et vers le trou transversal (10).

8. Clou médullaire selon l'une quelconque des revendications 6 ou 7, **caractérisé en ce que** les mâchoires (12, 13) sont prévues de telle sorte que les parties externes (14b) de l'espace libre (14) sont plus larges que le moyen de verrouillage (9).

9. Clou médullaire selon l'une quelconque des revendications 6 à 8, **caractérisé**
**en ce que** les mâchoires (12, 13) possèdent une longueur (L1) essentiellement supérieure à celle des côtés (10a, 10b) du trou (10) les plus proches de l'espace libre (14) lorsqu'on regarde dans la direction longitudinale du clou médullaire (5), et/ou
**en ce que** les côtés internes des mâchoires (12, 13) définissent ou forment des angles avec l'axe longitudinal géométrique (L) du clou médullaire (5) inférieurs à ceux formés par les portions frontales des côtés (10a, 10b) du trou (10) les plus proches de l'espace libre (14).

10. Clou médullaire selon l'une quelconque des revendications 6 à 9, **caractérisé en ce que** les mâchoires (12, 13) possèdent des portions marginales externes chanfreinées (12a, 13a).

11. Clou médullaire selon l'une quelconque des revendications précédentes, **caractérisé en ce que** le moyen de verrouillage (9) est une vis de verrouillage qui est vissée dans le trou pratiqué dans un fragment d'os (4) et qui peut être retirée ou extraite du trou transversal (10) pratiqué dans les parties frontales (5b) du clou médullaire (5) en la dévissant ou en la chassant hors dudit trou pratiqué dans le fragment d'os (4).

12. Clou médullaire selon l'une quelconque des revendications précédentes, **caractérisé**
**en ce que** les parties frontales (5b) du clou médullaire (5) possèdent un deuxième trou transversal (15) derrière ledit premier trou transversal (10) ; et en ce que le moyen de verrouillage transversal (9) possède un trou (16) ou un autre membre de fixation pour y fixer un accessoire, ledit accessoire étant conçu pour guider un foret afin de forer un deuxième trou dans un fragment d'os inférieur (4) pour un deuxième moyen de verrouillage transversal qui est conçu pour s'étendre à travers le deuxième trou transversal (15) du clou médullaire (5).

13. Clou médullaire selon l'une quelconque des revendications précédentes, **caractérisé en ce que** ledit clou médullaire (5) est conçu pour être utilisé pour des os de l'avant-bras, par exemple pour l'ulna.
